# EUROPEAN PATENT SPECIFICATION

(11) **EP 1 759 625 B1**
(45) Date of publication and mention of the grant of the patent: **29.10.2008**
(21) Application number: 05781667.0
(22) Date of filing: 18.08.2005
(51) Int. Cl.: A61B 1/00

(54) **A ENDOSCOPE ASSEMBLY WITH A DISPOSABLE SHEATH**
ENDOSKOP-ANORDNUNG MIT EINWEGSCHLEUSE
ASSEMBLAGE D'ENDOSCOPIE AVEC ETUI JETABLE

(30) Priority: 14.12.2004 CN 200420055751
(43) Date of publication of application: 07.03.2007
(73) Proprietor: Jiang, Kerang, Shenyang, Liaoning 110044 (CN); Jiang, Shoumei, Shenyang, Liaoning 110044 (CN)
(72) Inventor: Jiang, Kerang, Shenyang, Liaoning 110044 (CN); Jiang, Shoumei, Shenyang, Liaoning 110044 (CN)
(74) Representative: Stuart, Ian Alexander
(86) International application number: PCT/CN2005/001281
(87) International publication number: WO 2006/063497

(56) References cited:
- CN-A- 1 486 666
- CN-A- 1 486 667
- JP-A- 2004 129 813
- US-A- 4 741 326
- US-A- 5 050 585
- US-A- 5 386 817
- US-A- 5 643 175
- US-A- 5 746 694
- US-A1- 2004 077 927

## Description

### TECHNICAL FIELD

This invention relates to endoscope and more particularly to an endoscope system with a disposal sheath for effective isolation of an endoscope from contamination on endoscopy, it is an improvement on traditional medical endoscopes to solve disposal sterilization, belongs to medical instrument field, suited for clinical use in hospitals.

### BACKGROUND OF THE INVENTION

Medical endoscope is used for diagnostic and therapeutic purpose during Micro-invasive surgery due to its incomparable function, because the morpho-characters and excision of focus can be directly observed and performed on endoscopy. But the terrible problem is medical cross-infection in particular some virus infection, such as Hepatitis and AIDS, via blood or other body fluid by endoscopy. Therefore, disposable small medical instruments should be recommended. However endoscopes as an interfering instrument are quite high cost at present. People have not found any excellent way to effect and conveniently sterilize and prevent endoscopes from damage simultaneously. By now autoclaved method is recommended for some rigid, endoscopes' sterilization due to application of some optical glues which could bear high temperature and high pressure. But flexible endoscopes can't bear with autoclaved way, also disinfection solution can't thoroughgoing flow into varied channels to sterilize wholly. Since ninety decade last century, some foreign patents discussed the difficult issues thereinbefore. They generally set a disposal sheath on outside endoscope and transform endoscope body (this patent's applicant, JIANG, Kerang, also provides some patent applications numbered 03136141.2 (publication number CN-A-1486 667) and 02158217.3 et al), and disposal channel is settled on outside of the endoscope channel. The US patent numbered 5050585, (forming the preamble of claim 1) provides that of the disposal channel settled in inside of traditional endoscopes, and the anterior opening of disposal channel connected with the disposal sheath which covers on the endoscope to protect endoscope's outer surface and inner surface of opened channel, that is a good idea. Japanese Pentax company on this basis applied lots of patents for improvement later on. However, as to information from applications' publication, the cross-infection problem of medical endoscope in clinic application has yet not solved. In order to produce this kind of product, it will have yet a quite long way to go. More details are also needed to improve, such as fluid-air channel's position in endoscope system and other located connecting structure and correctly treat the disposal channel after use etc. All of such problems must be solved by new way.

### SUMMARY OF THE INVENTION

This invention provides a safe matching assembly system with an endoscope disposal sheath, to solve medical cross-infection thoroughly. That is to put forward newly program, improve lots of details, and reach clinical practice. The invention is defined in claim 1.

For protecting an improved permanent endoscope, the innovative uses a disposal sheath to ensure the endoscope not contaminant in clinic application. The said disposal sheath consists of a end cap, a capsule, a disposal channel, a soft double cavity fluid-air channel, a jet channel, a locking ring. Said end cap connects the disposal capsule which located in outside endoscope and the disposal channel which located in the endoscope channel and the soft double cavity fluid-air channel, thus all together connect with and join in a whole body, then to protect the endoscope's outer surface and channel's inner surface as well. The end cap is made from some elastic or partial elastic transparent materials, its inner end face's size fits in the end face's size of anterior endoscope. The end cap's inner face matches the endoscope body. A jet channel orifice is coaxially set at the anterior end cap to fix the jet channel. The space between the ditch and the channel need to tight glue with certain adhesive. A fixing seat or a fixing orifice of the disposal channel is set at the endoscope's operation orifice's position in the end surface of the end cap for convenient connecting with the disposal channel. The outer surrounding of posterior end cap is sheathed in the anterior end of the capsule. The capsule is made from elastic flexible thin wall nonpoisonous, non-immune biocompatible and lubricant or lubricated, medical material. The capsule covers the outside of endoscope. The posterior capsule connects a locking ring. While pull to lengthen, the elastic capsule can be locked by the locking ring to ensure the anterior inner face of the end cap constantly and tight close to anterior end face of the endoscope. Since no space between the capsule end cap end anterior tip of the endoscope, reflected complicated light formed by lighting ray at observation system can be decreased. The upper and lower handles of the locking ring have corresponding oblique teeth to tighten or loose the posterior capsule. The fluid-air channel is connected to posterior end of the jet channel, and located in between the endoscope and the capsule, the fluid-air channel can be a single cavity or double cavities, or the structure of two single cavity channels together join in one cavity channel at nearing jet channel site, its channel's size can be cylinder, or flat orifice, said the structure of single cavity channels, may be two flat channels which is formed via heat pressure, and parallel together with each other thin wall channels. The flat channels is not easy to be bent and to block the flowing fluid-air also can be inflated and expanded into two cylinder channel cavities while flowing fluid-air flow into them, said the structure of double cavity fluid-air channel, Inside of the anterior part of it which sheathes on the jet channel has not any septum, but inside the later part of the double cavity channel has a septum, and at the back of the jet channel anterior septum has a round head which fits in the inner wall of the channel cavity, and there is a section of incision, respectively at situation of the septum's two sides, separated from the channel wall in order to make the septum swing towards any side of channel cavity to block the channel which the flowing fluid needs not flow into; The posterior soft double cavity channel(including two channels) is sheathed on two rigid connecting tubes that is has two parallel channels which are welded together, the posterior orifice of two rigid connecting tube are sheathed in two adapters of the two soft channels connecting with fluid and air channels respectively. The jet of the jet channel which is connected with the anterior double cavity channel, sprays out the fluid or air and direct towards the outer surface of the end cap and fits in the object lens situated inside of the end cap. The disposal channel is made of flexible, durable, hard to bent, bear negative pressure and electric arc-resistant medical materials, its anterior tip connects with the end cap. Its connecting method includes to directly sheathe and glue the disposal channel on the end cap by set a channel seat or to sheathe by a channel seat which can bear high temperature and be electric arc-resistant, glued in fixing orifice of the end cap.

For the use of matching the disposal sheath, the endoscope body needs to be improved as follows:
① The original anterior endoscope's fluid-air channel and jet channel are removed, and placed into the position outside of the endoscope, only a fluid exit and a air exit are set on posterior endoscope below the handle. A sucking channel adapter is also set near the fluid-air exits mentioned above.
② The diameter of operation channel(endoscope channel) is enlarged properly, for being convenient to insert disposal channel into it.

In addition, this invention also may use a kind of three-way sealing cap, a guide tube, a sucking tube and two kinds of heat fusion forceps which can shear disposal channel to V-shape cut edge. This can isolate the endoscope channel from the contaminated disposal channel.

The three-way sealing cap is made from elastic material, the straight cavity is used for fixing and sealing the posterior end of the disposal channel and used as entrance or exit of instrument, its anterior end can be fixed on the exit of the endoscope channel, and on one side of the straight cavity set a sucking channel adapter used as a sucking way connecting the sucking tube via the disposal channel. Three elastic sealing orifices are set in the straight cavity. The inner diameter of three elastic sealing orifices is less than the outer diameter of the disposal channel. In order to solve the difficulty of inserting the disposal channel, the invention provides a kind of guide tube. The inner diameter of the guide tube is more than the outer diameter of the disposal channel. The outer diameter of the guide tube is more than the inner diameter of three sealing orifices of the three-way sealing cap. Due to the guide tube is made of rigid thin wall of tube, it can be, in advance, inserted into three-way sealing cap as to put the disposal channel into it with convenience. After the three-way sealing cap was fixed on, the guide tube is pulled out, then the disposal channel is kept in the three-way sealing cap. In order to let the disposal channel is correctly put in the exit of the second elastic sealing orifice, a tube core is set in the guide tube and the over-length part which is heightened than the sucking channel orifice can be pushed to the above mentioned application position by the lower end of the tube core. The inserting deepness of the guide tube core depends on the distance between the handle limiting face and orientation pin. The orientation pin is pushed to in front of the third elastic sealing orifice by the posterior end of the guide tube's long orifice, and to be blocked, during the process of pulling the guide tube out although the lower end of the guide tube leave away from the first elastic sealing orifice, the disposal channel, instantly is held by the first elastic sealing orifice, cannot turn over to raise till the guide tube bring the tube core continuous pulled out so that the disposal channel again be blocked in the second elastic sealing orifice of the three-way sealing cap and be then sealed and be keep-held, till the guide tube is pulled out wholly.

While the disposal sheath and the endoscope being in use, the first thing is to insert the disposal channel into the endoscope channel, later the other part of the disposal sheath is sheathed into it respectively. For solving the difficulty of inserting the disposal channel, to begin with, it is about to put an endoscope instrument, such as biopsy forceps into the endoscope channel, besides, to cover the instrument's head part by using the disposal channel and stretch out the endoscope head. While the instrument is in fixing position, not to remove, the disposal channel can be smoothly passed into the endoscope channel along a space between the endoscope instrument and endoscope channel. Then outside of endoscope can also be covered on by another part of the disposal sheath. After that, to pull upwards the locking ring to nearing handle position on endoscope body, then to firmly lock it. Thus the capsule's elastic thin membrane makes the end cap's inner end and anterior endoscope end's surface tight close with each other. There is clear vision field so far. At that time posterior end of the disposal channel already extends to exterior of the endoscope channel exit, the anterior end orifice of the guide tube which was inserted in the anterior three-way sealing cap sheathes on outside of posterior end orifice of the disposal channel, sheathes forwards and the three-way sealing cap is fixed on the endoscope channel exit site and the sucking channel connecting with the three-way sealing cap is connected to the sucking channel adapter, it can be used in clinic application after pulling out the guide tube when finishing this process , and two channels (fluid channel and air channel) are connected to two exit (fluid exit and air exit) of the endoscope respectively.

After the clinic use, the three-way sealing cap and the sucking channel need to pull out(the three-way sealing cap and the sucking channel are made of high temperature resistant material so that they can be reused after cleaning and disinfecting, of course also can be disposal), then the disposal sheath need to pull out Due to the disposal channel orifice has been contaminative, the outlet of the disposal channel must be well blocked. After be blocked and then extracted, this can avoid the endoscope channel from pollution. The endoscope channel can be protected and prevented from becoming re-polluted source in the later use. Two kinds of heat fusion forceps for cutting the outlet of the disposal channel and fusing certain shape of the disposal channel at the position nearing the posterior outlet of the disposal channel may be used. This shall ensure sheared the disposal channel under certain sterilization temperature when exiting. While fused and melted simultaneous the disposal channel to be cut out. The cut edge's size also need to meet certain requirement, that is while the sheared outlet of the disposal channel passed the endoscope channel, the cut-edge's shape becomes a V-shape, the disposal channel's shape still all is a full cylindrical not to be squeezed and opened, the cut edge's shape needs to become a V-shape. Thus the outlet of the disposal channel is in favor of not reopening. This invention may provide two kinds of V-shape cut edge which can satisfy the goal. Structure of the one of the heat fusion forceps is to set a piece of electric resistance thread in axial direction with V-shape at the forceps exit plane (the V-shape electric resistance thread also can be installed in contradirectional position), to set a flexible insulation board at an opposite plane's piece which can freely swing. The two planes are parallel with each other and can be freely closed. The second kind of heat fusion forceps has two pieces structures, piece-upper and piece-lower with a same degree, formed positive and negative V-shapes forceps exit. One piece can be moved radially and swing relatively for parallel pressurization to the channel exit. After heat the electric thread, the sheared outlet of the disposal channel can be changed into V-shape in radial direction as the first kind of heat fusion forceps also can achieve the same effectiveness. Compare to present technology, this invention has following advantages:
□ Both US 2004/0077927A1 and Japanese 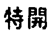 2004-129813 patents offered a tight turn method to shorten the space between capsule end cap and anterior end of endoscope in order to decrease reflected complicated light. But turning space of the endoscope channel certainly increased, so that it occupies precious radial space in endoscope. In this invention we use elasticity of the sheath to made elastic tight locking the protrusion platform of end cap, and saved the occupying space.
□ Japanese patent application, 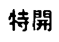 2003-153848, provides a method to block the disposal channel before removing from the endoscope channel after use. That method uses a pair positive-negative of C-shape R metal block to heat and melt the disposal channel and then cut the disposal channel by using a knife. Its inconvenience is that the positive-negative of C-shape R Ratio changes a lot depending on the thickness of the heated channel wall. This invention may provide two kinds of V-shape heat fusion forceps, which are easy and convenient during the operation, and the V-shape's angle does not relate to channel wall's thickness.
③Compared with other aspects of present technology, the invention also provides more advantages such as the three-way sealing cap's structure, a special guide tube and traction forceps different kind of fluid-air channel and its septum, and the anterior part of the septum can swing to avoid the back-flow function etc. It also has unique advantage and special points, to arouse elastic sealing cap smoothly fixing in the disposal channel.

### BREIF DESCRIPTION OF THE DRAWINGS

Fig. 1 is a whole body's appearance of this invention.
Fig. 2 shows the structure figure of a disposal sheath.
Fig. 3 shows the double cavity fluid-air channel.
Fig. 4 is the overlooking figure of the figure3.
Fig. 5 is a fluid-air channel consists of two flat channels.
Fig. 6 is a head part's structure figure of the disposal sheath.
Fig. 7 shows another kind of head part's structure figure of disposal sheath.
Fig. 8 is a head part's (having thin membrane) structure figure of disposal sheath.
Fig. 9 show is a locking ring.
Fig. 10 is a three-way sealing cap figure.
Fig. 11 is a guide tube figure.
Fig. 12 shows a guide tube inserting into three way sealing cap and a disposal channel for preparation to sheathe and shows endoscope channel outlet.
Fig. 13 shows a disposal channel is already sheathed into three way sealing cap and a disposal channel.
Fig. 14 shows preparation of pulling out the guide tube after the process of figure 13.
Fig. 15 shows a kind of V-shape heat fusion forceps.
Fig. 16 shows a section view of a V-shape heat fusion forceps at closing forceps' position as shown in figure 15.
Fig. 17 shows another example of the structure of V-shape head fusion forceps.
Fig. 18 shows two samples of a disposal channel's outlet have been cut off by a forceps as shown in figure 17.
Fig.19 shows a structure figure of a traction forceps.
Fig.20 is a magnified figure of head part of the traction forceps in figure 19.
In figures:1 is an endoscope, 2 is an end cap, 3 is a disposal channel, 3.1 is a channel seat 3.2 is a V-shape channel orifice, 3.3 is a other V-shape channel, 4 is a fluid-air channel,4.1 is a septum cavity,4.2 is septum of round head,4.3 is double channels adapters, 4.4 is connecting material of double channels adapters, 4.5 is rigid curved channel, 4.6 is single cavity channel, R1 is curve rate of channel cavity , R2 is curve rate of septum end head, 5 is a jet channel, 6 is a fluid-air channel adapter, 7 is a fluid-air exit, 8 is a capsule, 9 is a locking ring, 10 is an endoscopes channel, 11 is an endoscope channel exit,12 is a three-way sealing cap,12.1 is the first elastic sealing orifice,12.2 is the second elastic sealing orifice, 12.3 is the third elastic sealing orifice, 12.4 is a lower adapter, 12.5 is a sucking channel adapter, 12.6 is an upper orifice, 13 is a guide tube, 13.1 is a guide tube core, 13.2 is a handle, 13.3 is an orientation pin, 13.4 is a long orifice, 13.5 is a lower orifice of guide tube, 13.6 is an end face of handle,13.7 is a lower end of tube core, 13.8 is a tube body, 14 is a kind of heat fusion forceps, 14.1 is a lower forceps orifice, 14.2 is an electric resistance thread, 14.3 is an upper forceps orifice, 14.4 is a wire, 15 is an another kind of heat fusion forceps,15.1 active board 16 is a sucking channel adapter 17 is A sucking channel.

### DESCRIPTION OF THE INVENTION IN DETAIL

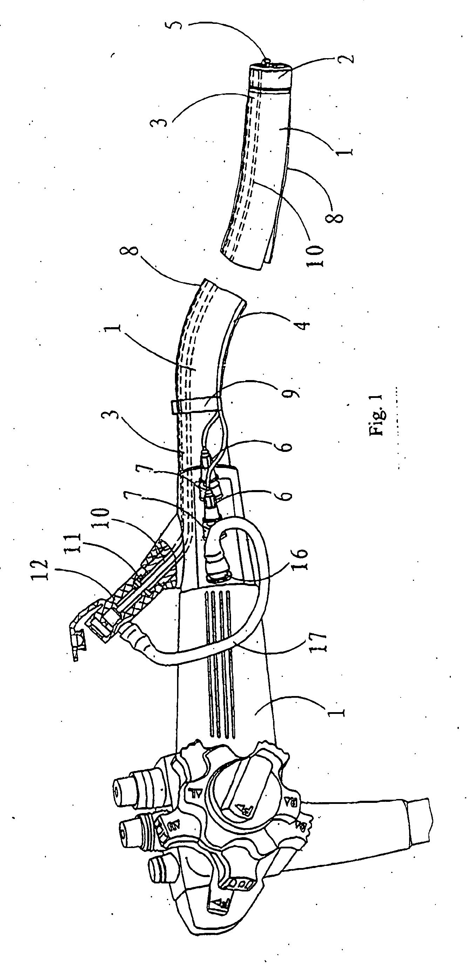

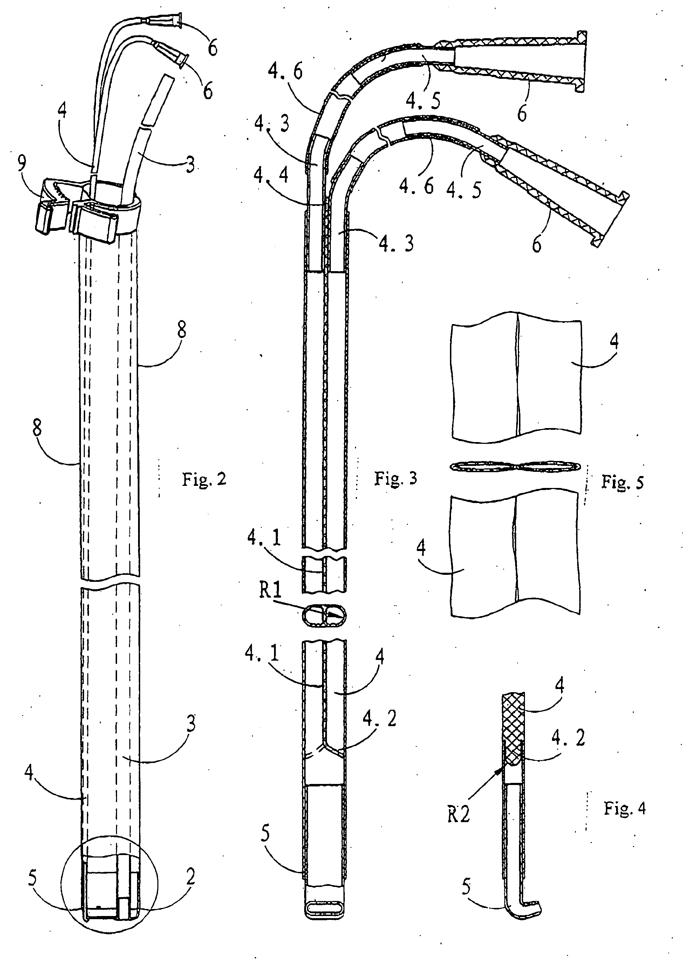

This invention consists of two parts, an improved permanent endoscope and a disposal sheath, altogether formed an endoscope system with the disposal sheath (see Fig.1). It offers the permanent endoscope, which can be used for many times, to be protected by the disposal sheath from pollution. The disposal sheath (see fig.2) not only has the capsule 8 and the end cap 2, to protect the endoscope louter-surface, but also has a disposal channel 3 placed into the endoscope channel 10 to protect the endoscope channel's inner-surface. The capsule and the disposal channel are connected through the end cap 2. And they are joined in a whole body, therefore, the endoscope of both inside and outside are protected in all.

The detail of above-mentioned endoscope system with a disposal sheath is as follows:

At first, to reform the traditional endoscope below:

In the anterior endoscope 1, there is not any fluid-air channel or jet channel. The fluid-air channel and the jet channel are removed to the outside of the endoscope position between the endoscope 1 and the capsule 8. The jet channel is fixed in the jet channel ditch which located in one side of the end cap, the fluid-air exit 7 and the sucking channel adapter 16 are set on the posterior endoscope. The fluid-air exit 7 and the sucking channel adapter 16 connect the fluid-air channel adapter 6 and the sucking channel 17 respectively. The sucking channel adapter 12.5 of the three-way sealing cap 12 sheathed with another end of the sucking channel 17.

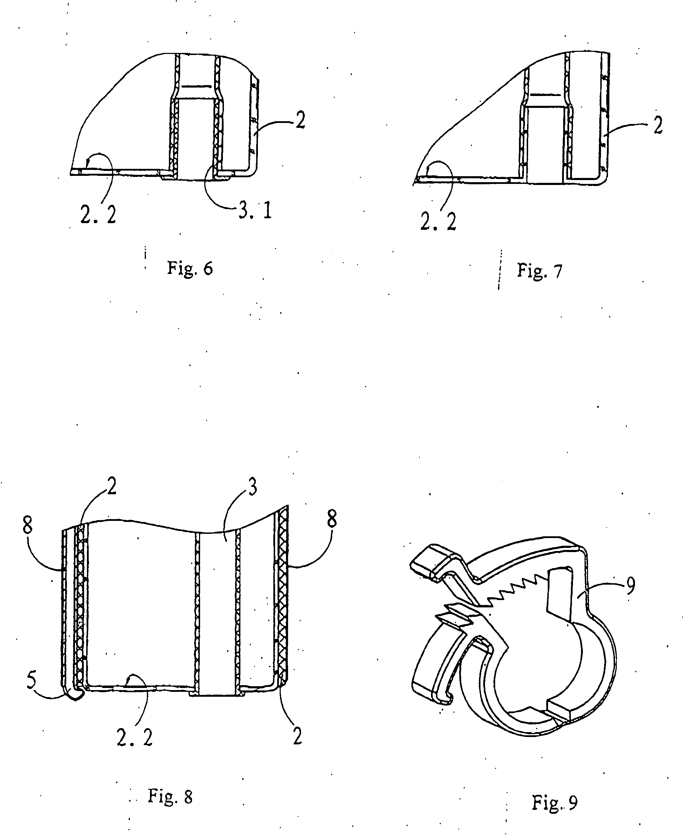

On the other aspect, the disposal sheath consists of the end cap2, the capsule 8, the disposal channel 3, the locking ring 9, the fluid-air channel 4, the jet channel 5, the fluid-air channel adapter 6, the three-way sealing cap12, the sucking channel 17 and guide tube 13 etc. The invention preferably also provides the traction forceps and two kinds of heat fusion forceps 14 and 15 for simultaneous melting and cutting the disposal channel 3 after the use, the end cap 2 is made of transparent or partial flexible transparent material. To decrease the complication light, out of illumination outgoing light and reflect into observation system, this invention preferably provides two kinds of program: due to conventional endoscopes' anterior end surface are not flat, and have different size, the invention offers the end cap as shown in figure 8. The end surface of the end cap is made of elastic, flexible, thin membrane, so that it can close to the illumination out-going light and the observation system while it is pulled and tightened and the complication light reflected between them can be decreased. For this reason, the invention preferably provides special endoscope-its anterior end surface is quite flat. As shown in figure 6 and figure 7 a kind of end cap, improved by this invention, processes its flat anterior inner surface. This makes closely contact possible between anterior end surface of endoscope and inner end surface of the end cap 2 which can decrease complication light as well. The posterior end of the jet channel 5 is connected with the fluid-air channel. The jet orifice of the jet channel 5 directs towards, outer end surface of the end cap relative to the object lens' position, and buckle up in the through ditch 2.3, its posterior end sheathes the fluid-air channel 4 the exit of the jet channel 5 directs towards the outer surface of the end cap relative to the object lens site. Anterior end cap, similar with anterior end of the endoscope, at the position of endoscope channel, opening the end surface of end cap 2 connects with the disposal channel 3, there are three connection ways:1) as shown in Fig 6, adopting the channel seat 3.1 to connect the end cap 2 with adhesive; 2) as shown in Fig 7, the channel seat 3.1 and the end cap 2 formed a whole body; 3) as shown in Fig 8, without any channel seat, the tip of the disposal channel turned over outwards. The capsule tight covers the outside of end cap 2. The parts that the end cap 2 connected with including the capsule 8, the jet channel 5, the disposal channel seat 3.1, the disposal channel 3, all applied with medical adhesive.

The capsule 8 is made of lacto rubber thin membrane material, or PVC thin membrane tube or polyaminoesters, or other high molecular material etc. Anterior end of the capsule 8 adhesively connects with the outside of posterior end cap 2 or majority of the outside of end cap 2. The length and diameter of the capsule 8 matches the length and diameter of the endoscope body, its posterior end connects to the locking ring 9. All of those connections are adhered with adhesive.

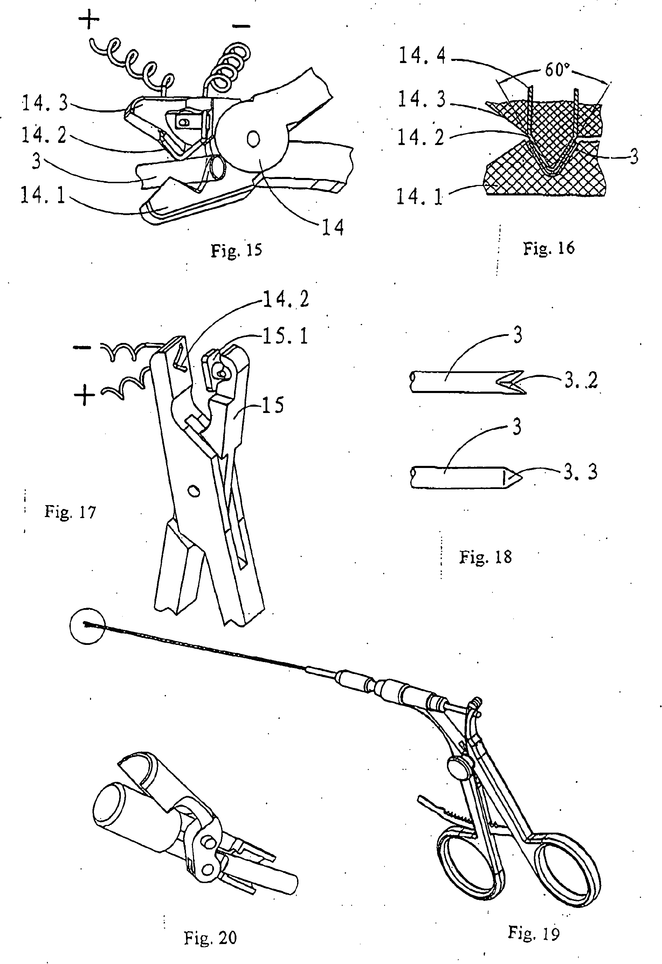

The disposal channel 3 is made from sturdy, elastic, high temperature resistance, electric arc resistance and bear negative pressure material, its length and diameter matches the length and diameter of the endoscope channel. Its anterior end is adhered on the end surface of the end cap 2, sites in the endoscope channel. In order to solve the inserting difficulty of the disposal channel 3, the operation can be: firstly to insert an instrument such as biopsy forceps into the endoscope channel 10, let the instrument's head surpass anterior end of endoscope channel, then outside of the instrument sheathes in the disposal channel, they are inserted into the endoscope channel 10 altogether. As shown in Fig.19 and Fig 20, a traction forceps can be used, if necessary, to pull the disposal channel 3 passing through the endoscope channel 10. The traction forceps has two pieces of forceps piece, one piece is a cylinder and other piece is a half bullet size for contacting most area of channel wall. The lock of the forceps handle has some locking teeth While the disposal channel 3 passes through the endoscope channel, the capsule 8 and the end cap 2 are pulled by the locking ring 9 and heightened. Two fluid-air channels adapters 6 sheathe on the fluid-air exit 7 of the endoscope. The fluid-air channel can be single cavity or double cavity channel or the structure of two single cavity channels are joined together in one channel at nearing jet channel site , the channels shape may be cylinders, or flat shape as shown in fig.5 the fluid-air channel consisted of two single cavity channels may be consisted of two flat channels, which is formed via heat pressure, and parallel together with each other. The advantage of the flat channel is can not bent and can not be blocked by the flowing fluid-air, in addition, the closing channel which is not entered into by the flowing fluid-air only occupies less space, as shown in fig 3, 4) said of the structure of two fluid-air channel 4, it has not any septum inside the sheathed part with the jet channel 5. but inside the later part of the double cavity channel has a septum cavity 4.1 and on the anterior septum part of the double cavity channel at the back of the jet channel swinging set a septum of round head 4.2, its curve rate of double cavity septum head R2 fits the curve rate of the channel cavity. There are two incisions separating from the cavity wall, located at two sides of the certain section of the septum of round head 4.2, the septum section of round head 4.2 can be swung towards the left or right, to block the one of two cavities of the channel which flowing fluid needs not to flow into, prevent injecting fluid or air from back flowing into this one cavity. The posterior end of the soft double cavity channel sheathes on the rigid double channels adapters 4.3, between the double channels there is a connecting material (in this application example is a welding pad piece), the posterior end of double channels adapters sheathe in two single cavity channels 4.6, Which again sheath on two rigid curve channels 4.5 connecting the fluid-air channels adapters in order to convenience connect with two fluid-air exits of the endoscope.

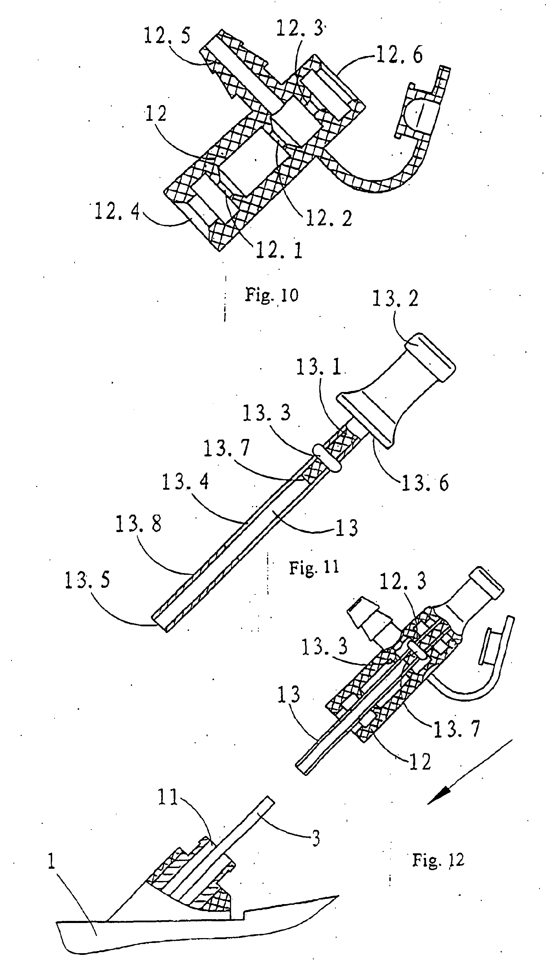

At that time, the part of the posterior end of disposal channel 3, which passed through the posterior end of the endoscope 11, needs to sheathe in a three-way sealing cap 12 as shown in Fig. 10. The straight cavity of the three-way sealing cap 12 has upper adapter 12.6and lower adapter 12.4. The first elastic sealing orifices 12.1, the second elastic sealing orifices 12.2, and the third elastic sealing orifices 12.3 are also set inside the straight cavity of the three-way sealing cap 12. At one side of the three-way sealing cap 12, the sucking channel adapter 12.5 is set for sheathing the sucking channel 17.The sucking channel 21connects with sucking channel adapter 16. On posterior endoscope, the sucking channel adapter20 connects with some relative channel or handy valve inside the endoscope. To suck the anterior disposal channel's fluid into fluid reserve by handy valve when needed.

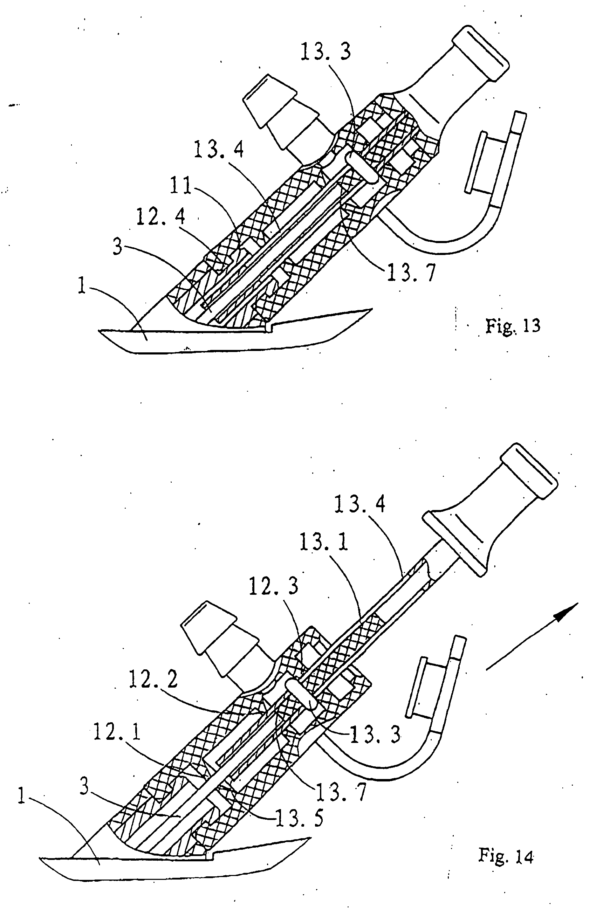

Owing to the inner diameter of the first to third elastic sealing orifices 12.1, 12.2, 12.3 of the three-way sealing cap 12 is less than the outer diameter of the disposal channel 3, for solve the inserting difficulty of the disposal channel 3, this invention preferably also provides the guide tube 13 as shown in Fig. 11. The guide tube 13 consists of a tube body 13.8 with a handle 13.2, a guide tube core 13.1, a long orifice 13.4, a orientation pin 13.3. The tube body 13.8 is made of thin wall tube. A certain length, symmetrical long orifice 13.4 is set at surface of its one side, to let the orientation pin 13.3 across the tube core 17.1 conveniently and slide in it. While match the guide tube with the three-way sealing cap(as shown in the fig.11) the distance between the handle end face 13.6 and posterior end of long orifice equals to the distance between the upper orifice of the three-way sealing cap and lower end face of the third elastic sealing orifice. The long orifice's length is more than the distance between the lower end of the guide tube and the first elastic sealing orifice of the three-way sealing cap. While the three-way sealing cap12 is prepared to sheathe in the disposal channel 3 and fix on endoscope channel exit, the followings are needed to be done: firstly to let the guide tube 13(as shown in Fig.12) sheathe in the three-way sealing cap 12, then to aim the lower end orifice 13.5 of the guide tube 13 at and cover the disposal channel orifice. While the three-way sealing cap 12 and sheathes on the endoscope (as shown in Fig.13) over length posterior end of disposal channel 3 is pressured to lower end of the sucking orifice by lower end of tube core 13.7. The orientation of the lower end of tube core 13.7 depends on the deepness decided by the orientation pin 13.3 and handle end face 13.6. As shown in Fig 12, the process of pulling the guide tube 13 out, at the time that lower orifice of guide tube 13.5 leaves the first elastic sealing orifice 12.1, the lower end of tube core 13.7 is yet locked on the front of the third elastic sealing orifice 12.3 due to the orientation pin. After the disposal channel 3 is contracted and fixed in the first elastic sealing orifice 12.1, till the guide tube 13 and tube core 13.1 are pulled out by the handle 13.2, as shown in Fig 14, the orientation pin 13.3 had retracted out of the third elastic sealing orifice 12.1, the lower end of tube core 13.7 no longer limit the disposal channel 3. The disposal channel is tight fixed in the first elastic sealing orifice, at that time again had tight fixed and sealed in the second elastic sealing orifice 12.2.

After clinic use, the disposal channel has contaminated all around, must be taken off treated as medical rubbish. Before the disposal channel 3 is taken out from the endoscope channel 10, the posterior end of channel orifice must be blocked, otherwise the endoscope channel 10 will be polluted. The blocking method is to ensure sterilizing the channel orifice and not reopen the channel orifice because of its bad size during the taking out process. Therefore, this invention may use two kinds of heat fusion forceps 14 and 15 (respectively as shown in Fig 15 and Fig 17). The disposal channel's orifice can be cut off and formed a V-shape by heating the electric resistance thread to one piece of two pieces of forceps exit. The cut-edge's temperature reaches sterilization condition. Said two forceps concrete structures are as follows: one is, as shown in Fig.15, the kind of heat fusion forceps 14, its channel orifice forms V-shape in radial direction V-shape electric resistance thread 14.2 in radial direction is parallel set on one piece of forceps opening. A cut edge by the heat fusion forceps of the disposal channel 3 formed a radial V-shape section as shown in Fig.16. The action of the forceps piece 14.3 makes the channel orifice balance and symmetric heating fusion; another heat fusion forceps 19 structured an axial direction V-shape of cut edge as shown in Fig.17. A movable insulation active board 15.1, set on one forceps piece, can form symmetric heat fusion. Its cutting edge forms a V-shape as shown in Fig.18. According to tests, the disposal channel 3 can basically be full cylindrical shape of channel for ensure after cutting off by this method, therefore, can be securely taken off from the endoscope channel 11 in favor of the blocked channel orifice not reopening. As shown in Fig.18, the disposal channel 3 formed an axial direction V-shape cut edge of channel-other V-shape channel 3.3 after cutting off by the heat fusion forceps (Fig.17). As shown in Fig. 17, the electric resistance thread 14.2 of the forceps orifice can be set and swung contradirectionally the same effeteness to the formed V-shape cut edge 3.2. Due to the three-way sealing cap 12 and the sucking channel 17 are made of materials which can bear high temperature, so that they can be reused after cleaning and disinfecting, of course also can be disposal. Since the sucking channel 13 did not contact with contamination at all, it can be cleaned by common method.

## Claims

1. An endoscope system comprising
(a) a reusable endoscope (1) including an endoscope channel member (10),
(b) a disposable sheath assembly including a sheath (8) for covering the exterior of the endoscope (1), a disposable channel member (3) for insertion into the endoscope channel member (10), and an end cap (2) which is joined with the disposable channel member (3) and the sheath (8) at the front ends thereof;
(c) a fluid-air channel member (4) having one or two through-passages, extending within the sheath (8) from adjacent the end cap (2) where it is coupled to a a jet channel (5), to a rear end region where it is coupled to a fluid-air channel adapter means (6);
(d) a three-way sealing cap (12) mounted at the rear of the endoscope (1), having a straight through passage (11) for passage of the disposable channel member (3), and a laterally-extending sucking channel adapter (12.5); and
(e) means for fusion-cutting the disposable channel member (3);
**characterised in that** the disposable sheath assembly includes the fluid-air channel member (4) and the fluid-air channel adapter means (6), and a locking-ring (9) embracing the sheath (8) at the rear end region thereof; said fluid-air channel member (4) extending externally of the endoscope (1);
**in that** said through passage (11) of the sealing cap (12) has three elastic sealing orifices (12.1,12.2, 12.3);
**in that** said means for fusion-cutting is heat fusion forceps adapted to melt and cut off the disposable channel member (3) to form a blocked V-shape;
and **in that** a guide tube (13) is provided which is insertable through the elastic sealing orifices (12.1,12.2, 12.3) of the through-passage (11) of the three-way sealing cap (12) from the rear, so that the disposable channel (3) can be passed through the guide tube (13) and hence through the sealing cap's through passage (11) from the front, whereafter the guide tube (13) can be withdrawn.

2. The endoscope system according to claim 1, **characterised in that** the end cap (2) is made of transparent material, its inner end face's shape coincides with the front end face of the endoscope (1); and the end cap (2) is embraced by the sheath (8).

3. The endoscope system according to claim 1 or claim 2, wherein the connection between the disposable channel member (3) and the end cap (2) is via a channel seat (3.1) provided on the end cap (2), or an adapter portion produced by turning the tip of the channel member over radially outwards.

4. The endoscope system according to claim 1, **characterised in that** the sheath (8) is made from elastic, flexible, thin-walled, non-poisonous, non-immunogenic, biocompatible and lubricant or lubricated material, its front portion covers the end cap (2); and the locking ring (9) has portions with mutually engagable oblique teeth and a handle whereby the locking ring can be adjusted to make the sheath (8) tighten or loosen about the endoscope, and can be locked up tight or unlocked by means of the handle.

5. The endoscope system according to claim 1, **characterised in that** the fluid-air channel member (4) has two through passages divided by a septum (4.1) except adjacent the end cap where there is no septum (4.1), and the channel member (4) engages over the jet channel (5) which is fast with the end cap (92); the septum (4.1) terminating in a round head with a curve rate the same as that of the channel cavity, the rounded head being cut free at each side so that it can swing in either direction, to block off either passage; the passages at the rear end of the member (4) engaging over respective rigid connecting tubes (4.3) with a welding pad piece (4,4) between them; the rear ends of said rigid connecting tubes (4.3) being connected to two fluid-air channel adapters constituting said fluid-air channel adapter means (6).

## Patentansprüche

1. Endoskopsystem, umfassend
(a) ein wiederverwendbares Endoskop (1), das ein Endoskopkanalelement (10) umfasst,
(b) eine Einweg-Hüllenanordnung, umfassend eine Hülle (8) zum Bedecken des äußeren Teils des Endoskops (1), ein Einweg-Kanalelement (3) zur Einbringung in das Endoskop-Kanalelement (10), sowie eine Endkappe (2), die mit dem Einweg-Kanalelement (3) und der Hülle (8) an den vorderen Enden derselben verbunden ist;
(c) ein Fluid-Luft-Kanalelement (4) mit einem oder zwei Durchtritts-Durchlässen, die sich innerhalb der Hülle (8) von der Umgebung neben der Endkappe (2), wo dieses an einen Düsenkanal (5) gekoppelt ist, bis zu einem hinteren Endbereich wegerstrecken, wo dieses an ein Fluid-Luft-Kanal-Adaptermittel gekoppelt ist (6);
(d) eine Dreiweg-Dichtungskappe (12), die auf der Rückseite des Endoskops (1) befestigt ist, die einen geraden Durchtritts-Durchlass (11) zum Durchtritt des Einweg-Kanalelements (3) und einen sich seitlich erstreckenden Saugkanaladapter (12.5) aufweist; sowie
(e) ein Mittel zum Schmelzschneiden des Einweg-Kanalelements (3);
**dadurch gekennzeichnet, dass** die Einweg-Hüllenanordnung das Fluid-Luft-Kanalelement (4) und das Fluid-Luft-Kanal-Adaptermittel (6) und einen Klemmring (9) umfasst, der die Hülle (8) am hinteren Endbereich derselben umschließt; wobei sich das Fluid-Luft-Kanalelement (4) außerhalb des Endoskops (1) erstreckt,
dass der Druchtritts-Durchlass (11) der Dichtungskappe (12) drei elastische Dichtungsmündungen (12.1,12.2,12.3) aufweist,
dass das Mittel zum Schmelzschneiden eine Wärmeschmelz-Zange ist, die zum Schmelzen und Abschneiden des Einweg-Kanalelements (3) angepasst ist, um eine undurchdringbare V-Form zu bilden;
und dass ein Führungsrohr (13) bereitgestellt ist, das durch die elastischen Dichtungsmündungen (12.1, 12.2, 12.3) des Durchtritts-Durchlasses (11) der Dreiweg-Dichtungskappe (12) vom hinteren Teil einbringbar ist, so dass der Einweg-Kanal (3) durch das Führungsrohr (13) und daher durch den Durchtritts-Durchlass (11) der Dichtungskappe von der Vorderseite geführt werden kann, wonach das Führungsrohr (13) zurückgezogen werden kann.

2. Endoskopsystem nach Anspruch 1, **dadurch gekennzeichnet, dass** die Endkappe (2) aus transparentem Material hergestellt ist, die Form der inneren Stirnseite mit der vorderen Stirnseite des Endoskops (1) übereinstimmt (1), sowie die Endkappe (2) von der Hülle (8) umschlossen ist.

3. Endoskopsystem nach Anspruch 1 oder Anspruch 2, worin die Verbindung zwischen dem Einweg-Kanalelement (3) und der Endkappe (2) durch eine Kanal-Auflage (3.1) besteht, die auf der Endkappe (2) oder einem Adapter-Abschnitt bereitgestellt ist, der durch das radiale Umdrehen des Endstücks des Kanalelements nach außen erzielt wird.

4. Endoskopsystem nach Anspruch 1, **dadurch gekennzeichnet, dass** die Hülle (8) aus einem elastischen, flexiblen, dünnwandigen, ungiftigen, nicht immunogenen, biokompatiblen und schmierenden oder geschmierten Material besteht, ihr vorderer Abschnitt die Endkappe (2) bedeckt; und der Klemmring (9) Abschnitte mit wechselseitig ineinander einrastbaren schrägen Zähnen und einem Griff hat, wobei der Klemmring angepasst werden kann, um die Hülle (8) um das Endoskop enger oder weiter zu stellen, und durch den Griff eng geschlossen oder geöffnet werden kann.

5. Endoskopsystem nach Anspruch 1, **dadurch gekennzeichnet, dass** das Fluid-Luft-Kanalelement (4) zwei Durchtrifts-Durchlässe aufweist, die - mit Ausnahme der Umgebung der Endkappe, wo es kein Septum (4.1) gibt - durch ein Septum (4.1) getrennt sind, und das Kanalelement (4) um den Düsenkanal (5) herum eingreift, der an der Endkappe (92) befestigt ist; wobei das Septum (4.1) in einer runden Kopfform mit derselben Krümmungsrate wie jene des Kanalhohlraums endet, der gerundete Kopf an jeder Seite freigeschnitten ist, so dass er in jede Richtung schwingen kann, um beide Durchlässe zu blockieren; wobei die Durchlässe am hinteren Ende des Elements (4) um jeweilige starre Verbindungsrohre (4.3) herum mit einem Schweißkissenelement (4.4) zwischen ihnen eingreifen; und wobei die hinteren Enden der starren Verbindungsrohre (4.3) mit zwei Fluid-Luft-Kanal-Adaptem verbunden sind, welche das Fluld-Luft-Kanal-Adaptermittel (6) ausbilden.

## Revendications

1. Système d'endoscope comprenant
(a) un endoscope réutilisable (a) incluant un élément de canal d'endoscope (10) ;
(b) un ensemble de gaine jetable incluant une gaine (8) pour couvrir l'extérieur de l'endoscope (1), un élément de canal jetable (3) pour l'insertion dans l'élément de canal d'endoscope (10), et un capuchon d'extrémité (2) qui est relié à l'élément de canal jetable (3) et à la gaine (8) à leurs extrémités frontales ;
(c) un élément de canal fluide-air (4) ayant un ou deux passages traversants, s'étendant dans la gaine (8) depuis le voisinage du capuchon d'extrémité (2) où il est relié à un canal à jet (5), à une région d'extrémité arrière où il est relié à un moyen d'adaptateur de canal fluide-air (6) ;
(d) un capuchon d'étanchéité (12) à trois voies monté sur l'arrière de l'endoscope (1), ayant un passage traversant rectiligne (11) pour le passage de l'élément de canal jetable (3), et un adaptateur de canal d'aspiration (12.5) s'étendant latéralement ; et
(e) un moyen de fusion-coupe de l'élément de canal jetable (3) ;
**caractérisé en ce que** l'ensemble de gaine jetable comprend un élément de canal fluide-air (4) et le moyen d'adaptateur de canal fluide-air (6) et une bague de verrouillage (9) entourant la gaine (8) à sa région d'extrémité arriéré ; ledit élément de canal fluide-air (4) s'étendant à l'extérieur de l'endoscope (1) ;
**en ce que** ledit passage traversant (11) du capuchon d'étanchéité (12) possède trois orifices d'étanchéité élastiques (12.1, 12.2, 12.3) ;
**en ce que** ledit moyen de fusion-coupe est un forceps de fusion thermique apte à faire fondre et à couper l'élément de canal jetable (3) pour former une forme en V bloquée ;
et **en ce qu'**un tube de guidage (13) est prévu qui peut être inséré à travers les orifices d'étanchéité élastiques (12.1, 12.2, 12.3) du passage traversant (11) du capuchon d'étanchéité à trois voies (12) depuis l'arrière de sorte que le canal jetable (3) peut être passé à travers le tube de guidage (13) et donc à travers le passage traversant (11) du capuchon d'étanchéité depuis l'avant, après quoi le tube de guidage (13) peut être retiré.

2. système d'endoscope selon la revendication 1, **caractérisé en ce que** le capuchon d"extrémité (2) est réalisé à partir d'un matériau transparent, sa forme de face d'extrémité intérieure coïncide avec la face d'extrémité avant de l'endoscope (1) et le capuchon d'extrémité (2) est entouré par la gaine (8).

3. Système d'endoscope selon la revendication 1 ou la revendication 2, où la connexion entre l'élément de canal jetable (3) et le capuchon d'extrémité (2) se fait par un siège de canal (3.1) réalisé sur le capuchon d'extrémité (2), ou bien une portion d'adaptateur produite en faisant tourner la pointe de l'élément de canal radialement vers l'extérieur.

4. Système d'endoscope selon la revendication 1, **caractérisé en ce que** la gaine (8) est réalisée à partir d'un matériau élastique, flexible, à paroi mince, non toxique, non-immunogène, biocompatible et lubrifiant ou lubrifié, sa portion avant couvre le capuchon d'extrémité (2) ; et la bague de verrouillage (9) possède des portions avec des dents obliques pouvant être mutuellement mises en prise et une poignée par laquelle la bague de verrouillage peut être ajustée pour resserrer ou relâcher la gaine (8) autour de l'endoscope et peut être verrouillée ou déverrouillée par la poignée.

5. Système d'endoscope selon la revendication 1, **caractérisé en ce que** l'élément de canal fluide-air (4) présente deux passages traversants divisés par un septum (4.1), excepté à proximité du capuchon d'extrémité où il n'y a pas de septum (4.1.), et l'élément de canal (4) s'engage sur le canal à jet (5) qui est solidaire avec le capuchon d'extrémité (92) ; le septum (4.1) se terminant par une tête ronde avec un taux de coupe qui est le même que celui de la cavité de canal, la tête arrondie étant coupée libre à chaque côté de sorte qu'elle peut pivoter dans l'une quelconque des directions pour bloquer l'un quelconque des passages ; les passages à l'extrémité arrière de l'élément (4) s'engageant sur des tubes de connexion rigides respectifs (4.3) avec une pièce de coussinet de soudage (4.4) entre eux ; les extrémités arrière desdits tubes de connexion rigides (4.3) étant reliées à deux adaptateurs de canal fluide-air formant ledit moyen d'adaptateur de canal fluide-air (6).
